Europäisches Patentamt

European Patent Office

Office européen des brevets

(11) Publication number: **0 231 513 B1**

# EUROPEAN PATENT SPECIFICATION

(45) Date of publication of patent specification: **09.10.91**  (51) Int. Cl.⁵: **A61M 16/01**

(21) Application number: **86118002.4**

(22) Date of filing: **23.12.86**

(54) Gasification and dosage arrangement.

(30) Priority: **21.01.86 SE 8600250**

(43) Date of publication of application:
**12.08.87 Bulletin 87/33**

(45) Publication of the grant of the patent:
**09.10.91 Bulletin 91/41**

(84) Designated Contracting States:
**BE CH DE ES FR GB IT LI NL**

(56) References cited:
**DE-A- 2 706 192      DE-A- 3 522 452
GB-A- 1 299 311      US-A- 1 483 620
US-A- 3 158 154      US-A- 3 703 172
US-A- 3 998 239**

(73) Proprietor: **GAMBRO ENGSTRÖM AB
Box 201 09
S-161 20 Bromma(SE)**

(72) Inventor: **Ankartross, Jan Olov
Kragstalundsvägen 9
S-186 00 Vallentuna(SE)**
Inventor: **Lundell, Ulf Gustav
Bergfinkvägen 27
S-140 32 Grödinge(SE)**
Inventor: **Nyman, Rune
Nybodagatan 7
S-171 42 Solna(SE)**

(74) Representative: **Boberg, Nils Gunnar Erik
Gambro AB Patent Department Box 10101
S-220 10 Lund(SE)**

## Description

### TECHNICAL FIELD

The present invention relates to a dosage and gasification arrangement, intended in particular for the feeding of anaesthetic gas to a fresh gas stream in an anaesthesia apparatus, comprising a source of gas of constant pressure, a source of a liquid which is intended for gasification, a gasification chamber and a dosage line, which is arranged to conduct the gas from the gasification chamber to a mixing point for mixture with another gas, e g the said fresh gas by means of a valve arrangement giving a defined dosage of gas per Stroke of a control valve of said valve arrangement.

It will be clear, however, to those versed in the art that such an arrangement can also be used for other purposes, e g for the moistening of breathing gases in a respirator apparatus.

### BACKGROUND ART

Various experiments have been carried out to gasify anaesthetic liquid or moistening liquid directly into the fresh gas stream. See, for example US patents 3 251 361, 4 038 980 and 4 657 008. In such systems it has proved difficult, however, to bring about a uniform and controllable dosage.

Experiments also have been carried out with gasification of anaesthetic liquid in a separate gasification chamber were both gas and liquid phase existed simultaneously. Here a problem arose, though, at the filling up of new liquid. This problem is solved in a simple manner with the help of the present invention (c.f. e.g. SE-A-8403447).

Reference is also being made to DE-A1-27 06 192 which is describing a device of essentially the above mentioned kind and as set out in the first part of claim 1 comprising a source of liquid which is fed to a gasification chamber. An important difference compared with the present invention is, however, that said gasification chamber is not adapted to be kept at a constant pressure at a predetermined temperature.

### DISCLOSURE OF INVENTION

The present invention thus relates to a gasification and dosage arrangement of the type defined above which, more particularly, is characterized in that the gasification chamber is adapted to be kept at a constant pressure and, by means of a controllable heating arrangement is adapted to be maintained at such temperature that the liquid fed from the source into the gasification chamber, in the main, is directly gasified and after equilibrium has been reached no more liquid can be introduced from the source into the chamber than is apportioned in the dosage line by means of the valve arrangement arranged in the dosage line. In this manner, after equilibrium has been reached, a uniform gas mass of a constant temperature and constant pressure is obtained from which smaller quantitites readily can be apportioned into another gas, e g a fresh gas, in an anaesthesia apparatus.

The said constant pressure preferably is arranged to be brought about by feeding the liquid which is to be gasified at the said pressure. This can be achieved in that the source of liquid is adapted to be maintained at this pressure by feeding a gas, e g air, under the same pressure from a source of pressure via a governor.

In order to achieve the dosage, the dosage line may be provided with a time-controlled valve, the opening frequency and/or opening time of which can be varied. Alternatively the dosage line may be provided instead with a, preferably controllable, throttle valve.

In practice it has been found to be appropriate to arrange the gasification chamber at a higher level than the source of liquid and connect it to the same via a riser which, starting from a point near the bottom of the source of liquid, preferably ends near the bottom of the gasification chamber. At the same time it may be suitable to let the dosage line originate from the gasification chamber at the highest point of the latter or near that point.

The invention can also be applied to the dosage of liquid in that a condensation stage is inserted directly after the abovementioned dosage valve. The dosage thus takes place in gaseous state, whereupon the gas is reformed to liquid. In this way a very precise method for the dosage, especially of small quantities of liquid is achieved, something which otherwise is very difficult.

### BRIEF DESCRIPTION OF DRAWINGS

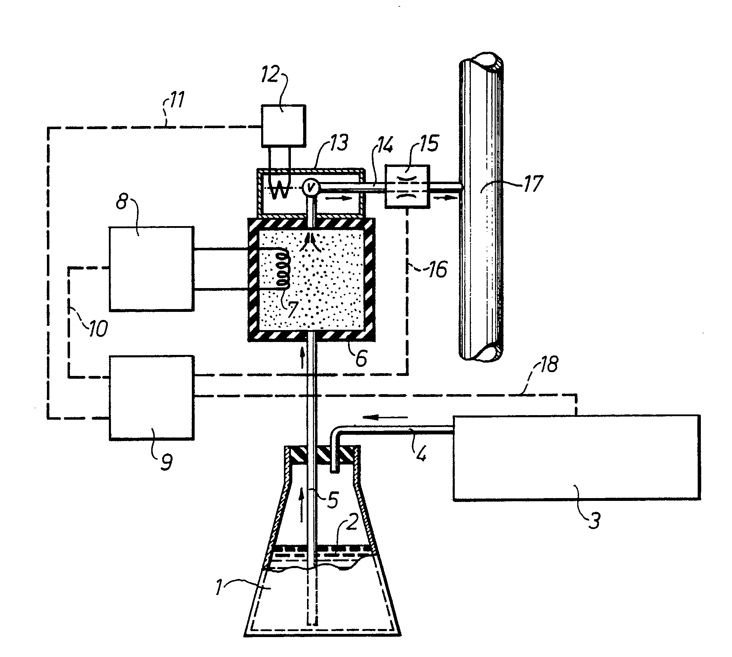

The invention will be described in greater detail in the following with reference to the attached drawing which, by way of example, schematically shows a preferred embodiment of the same.

### BEST MODE OF CARRYING OUT THE INVENTION

The preferred embodiment of the subject of the invention shown, by way of example, on the attached drawing is intended to be used in an anaesthesia apparatus. In the following description reference is made, therefore, to such an application, even though it will be clear to those versed in the art that the same principle can also be used for other purposes e.g., as stated above, to the dosage of liquid.

On the drawing is shown a source 1 for an anaesthesia liquid 2. In practice this source may be constituted of a liquid-filled pressure-proof glass flask which is intended to be supplied with a constant pressure via a governor from a source of pressure, these items being jointly designated 3. This may be done simply by means of feeding compressed air via the line 4. The liquid 2 is made thereby to rise via the riser pipe 5 up to a gasification chamber 6 which via a schematically indicated heating arrangement 7 is adapted to be maintained at such a temperature that the liquid fed, in the main, is directly gasified. The heating is controlled in the case shown by a controller device 8 which in turn is controlled by a microprocessor or the like 9. This is indicated by the broken line 10. A broken line 11 indicates how the same microprocessor 9 can control a controller device 12 for a schematically indicated solenoid valve 13 which controls the flow in a dosage line. This dosage line 14, moreover or alternatively, may contain a throttle valve 15 which possibly may also be controllable by the microprocessor 9, as indicated by the broken line 16. The dosage line 14 terminates in a fresh gas line 17, only a small secton of which is shown. Numeral 18 finally designates a broken line which indicates that the microprocessor also may be adapted to control the combination of governor and source of gas designated 3.

When it is a matter of the commonly occuring anaesthetic liquids, such as halothane, n-furan or isofuran, it has proved to be appropriate to make use of a temperature of approx. 70-80° C in the gasification chamber 6. At the same time it has proved appropriate to use a pressure of the order of magnitude of 400 cm water column, that is to say 0.4 bar. The pressure in the gasification chamber 6 hereby may not rise above 400 cm water column, since in such a case the liquid would be pressed back. Within the abovementioned temperature range the liquid gasifies directly as it enters the gasification chamber. At the same time no more liquid can be introduced that is apportioned via the dosage line 14.

In a practical embodiment a constant opening duration of the solenoid valve 13, namely 60 ms, is used, the opening frequency being varied between 0.1 Hz and 10 Hz. At the same time a throttle 15 is used here which was set so that it gave a dosage of 1 ml per stroke. As a result the dosage could readily be varied between 0.1 ml and 10 ml/s. Alternatively or moreover it also would have been possible to vary the opening duration. On using a solenoid valve this would have been possible from a minimum time of approx. 20 ms up to being kept open permanently.

The pressure too can be varied, of course, but is limited in practice by the sturdiness of the flasks wherein the anaesthetic gas is normally supplied in liquid form. The pressure is limited downwards by the fact that if the pressure is too low the liquid level in the vessel 1 would acquire a decisive importance for the dosage. In practice preferably a pressure between 100 and 800 cm water column is used.

Naturally the invention is not limited simply to the embodiment described above, but may be varied within the scope of the following claims. For example, the invention may also be applied to the dosage of liquid. In such an imaginary application the device 15 shown schematically may constitute a condensation apparatus wherein the quantity of liquid metered in gaseous state recovers its liquid form.

## Claims

1. A dosage and gasification arrangement, intended in particular for the feeding of anaesthetic gas to a fresh gas stream in an anaesthesia apparatus, comprising a source (3) of gas of constant pressure, a source (1) of a liquid which is intended for gasification, a gasification chamber (6) and a dosage line (14) which is arranged to conduct the gas from the gasification chamber (6) to a mixing point (17) for mixture with another gas, e g the said fresh gas and a valve arrangement (12,13,15) giving a defined dosage of gas per stroke of a control valve (13) of said valve arrangement, **characterized** in that the gasification chamber (6) is adapted to be kept at a constant pressure and, by means of a controllable heating arrangement (7) is adapted to be maintained (8,9,10) at such temperature that the liquid fed from the source (1) into the gasification chamber (6), in the main, is directly gasified and after equilibrium has been reached no more liquid can be introduced from the source (1) into the chamber (6) than is apportioned in the dosage line (14) by means of the valve arrangement (12,13,15) arranged in the dosage line (14).

2. An arrangement in accordance with claim 1, **characterized** in that the said source (3) of gas of constant pressure is arranged for keeping said source (1) of the liquid at said constant pressure in order to feed the liquid from the source (1) to the gasification chamber (6) at the same pressure.

3. An arrangement in accordance with claim 2, **characterized** in that the valve (13) provided in the dosage line (14) is time-controlled, the opening frequency and/or opening time of which can be varied.

4. An arrangement in accordance with claim 1 or 2, **characterized** in that the dosage line (14) is provided with a, preferably controllable, throttle valve (15).

5. An arrangement in accordance with anyone of the preceding claims, **characterized** in that the gasification chamber (6) is arranged at a higher level than the source of liquid (1) and is connected to the same via a riser (5) which, starting from a point near the bottom of the source of liquid, preferably ends near the bottom of the gasification chamber.

6. An arrangement in accordance with claim 5, **characterized** in that the dosage line (14) originates from the gasification chamber (6) at its highest point or near this point.

7. An arrangement in accordance with anyone of the preceding claims relating to the dosage of liquid, **characterized** in that the dosage take place in gaseous state, whereupon the gas is reformed to liquid.

**Revendications**

1. Dispositif de dosage et de gazéification, destiné en particulier à l'introduction de gaz anesthésiant dans un courant de gaz frais dans un appareil d'anesthésie, comprenant une source (13) de gaz sous pression constante, une source (1) d'un liquide qui doit être gazéifié, une chambre de gazéification (6) et une conduite de dosage (14) qui est prévue pour conduire le gaz de la chambre de gazéification (6) à un point de mélange (17) pour mélange avec un autre gaz, par exemple ledit gaz frais, et un agencement d'obturation (12, 13,15) fournissant une dose définie de gaz par course d'une vanne de commande (13) faisant partie dudit agencement d'obturation, caractérisé en ce que la chambre de gazéification (6) est prévue pour être maintenue sous une pression constante et, au moyen d'un dispositif de chauffage réglable (7), elle est prévue pour être maintenue (8,9,10) à une température telle que le liquide introduit à partir de la source (1) dans la chambre de gazéification (6) est, pour l'essentiel, directement gazéifié et, lorsqu'un équilibre a été atteint, on ne peut pas introduire plus de liquide, de la source (1) dans la chambre (6), qu'on en distribue dans la conduite de dosage (14) par l'intermédiaire de l'agencement d'obturation (12,13,15) placé dans la conduite de dosage (14).

2. Dispositif suivant la revendication 1, caractérisé en ce que ladite source (3) de gaz sous pression constante est prévue pour maintenir ladite source (1) du liquide à ladite pression constante afin d'amener le liquide de la source (1) à la chambre de gazéification (6) à la même pression.

3. Dispositif suivant la revendication 2, caractérisé en ce que la vanne (13) prévue dans la conduite de dosage (14) est commandée par minuterie, et on peut faire varier sa fréquence d'ouverture et/ou sa durée d'ouverture.

4. Dispositif suivant la revendication 1 ou 2, caractérisé en ce que la conduite de dosage (14) est pourvue d'une vanne d'étranglemnt (15), de préférence réglable.

5. Dispositif suivant l'une quelconque des revendications précédentes, caractérisé en ce que la chambre de gazéification (6) est placée à un niveau plus élevé que celui de la source de liquide (1) et elle est reliée à cette dernière par un tube montant (5) qui part d'un point proche du fond de la source de liquide et se termine de préférence près du fond de la chambre de gazéification.

6. Dispositif suivant la revendication 5, caractérisé en ce que la conduite de dosage (14) part de la chambre de gazéification (6) à son point le plus haut ou près de ce point.

7. Dispositif suivant l'une quelconque des revendications précédentes, destiné au dosage de liquide, caractérisé en ce que le dosage est effectué à l'état gazeux, après quoi le gaz est retransformé en liquide.

**Patentansprüche**

1. Dosier- und Vergasungseinrichtung, insbesondere für die Einführung von anästhetischem Gas in einen Frischgasstrom in einer Anästhesieapparatur, mit einer Quelle (3) für Gas von konstantem Druck, einer Quelle (1) für eine Flüssigkeit, die für die Vergasung bestimmt ist, einer Vergasungskammer (6) und einer Dosierleitung (14), die so angeordnet ist, daß sie das Gas aus der Vergasungskammer (6) zu einem Mischungspunkt (17) zum Vermischen mit dem anderen Gas, z. B. dem Frischgas, führt, und einer Ventileinrichtung (12, 13, 15), die eine definierte Gasdosierung je Stoß eines Steuerventils (13) der Ventileinrichtung ergibt, **dadurch gekennzeichnet,** daß die Vergasungskammer (6) so ausgebildet ist, daß sie auf einem konstanten Druck gehalten wird und mit Hilfe einer steuerbaren Erwärmungseinrichtung (7) so ausgebildet ist, daß sie auf einer solchen Temperatur gehalten wird (8, 9, 10), daß die aus der Quelle (1) in die Vergasungskammer (6) eingeführte Flüssigkeit im wesentlichen direkt vergast wird und nach Erreichen eines Gleichgewichts nicht mehr Flüssigkeit von der Quelle (1) in die Kammer (6) eingeführt werden kann, als in der Dosierleitung (14) mit Hilfe der Ventileinrichtung (12, 13, 15), die in der Dosierleitung (14) angeordnet ist, zugemessen wird.

2. Einrichtung nach Anspruch 1, **dadurch gekennzeichnet,** daß die Quelle (3) für Gas von konstantem Druck für ein Halten der Quelle (1) der Flüssigkeit auf diesem konstanten Druck ausgebildet ist, um die Flüssigkeit von der Quelle (1) zu der Vergasungskammer (6) mit dem gleichen Druck zu überführen.

3. Einrichtung nach Anspruch 2, **dadurch gekennzeichnet,** daß das in der Dosierleitung (14) vorgesehene Ventil (13) zeitlich so gesteuert wird, daß die Öffnungsfrequenz und/oder Öffnungszeit desselben variiert werden kann.

4. Einrichtung nach Anspruch 1 oder 2, **dadurch gekennzeichnet,** daß die Dosierleitung (14) mit einem vorzugsweise steuerbaren Drosselventil (15) versehen ist.

5. Einrichtung nach einem der vorausgehenden Ansprüche, **dadurch gekennzeichnet,** daß die Vergasungskammer (6) höher als die Flüssigkeitsquelle (1) angeordnet und mit dieser über ein Steigrohr (5) verbunden ist, welches, ausgehend von einem Punkt nahe dem Boden der Flüssigkeitsquelle, vorzugsweise nahe dem Boden der Vergasungskammer endet.

6. Einrichtung nach Anspruch 5, **dadurch gekennzeichnet,** daß die Dosierleitung (14) an der Vergasungskammer (6) an deren höchstem Punkt oder nahe diesem Punkt beginnt.

7. Einrichtung nach einem der vorausgehenden Ansprüche für die Flüssigkeitsdosierung, **dadurch gekennzeichnet,** daß die Dosierung in gasförmigem Zustand stattfindet, wonach das Gas in Flüssigkeit rücküberführt wird.